# EUROPEAN PATENT APPLICATION

(11) **EP 2 813 244 A2**
(43) Date of publication of application: **17.12.2014**
(21) Application number: 12866825.8
(22) Date of filing: 26.01.2012
(51) Int. Cl.: A61K 41/00

(54) **SIDE-EFFECT FREE DOSAGE FORM AND METHOD FOR PREPARING SAME**

(71) Applicant: Germanov, Evgeny Pavlovich, Moscow 129085 (RU); Chekhovskaya, Galina Stepanovna, 117593 Moscow (RU)
(72) Inventor: SURINOV, Boris Pavlovich, Obninsk Kaluzhskaya obl. 249030 (RU); KHACHUMOVA, Karine Georgievna, Moscow 109156 (RU)
(74) Representative: Jeck, Anton
(86) International application number: PCT/RU2012/000033
(87) International publication number: WO 2013/112066

(57) **Abstract**

The claimed group of inventions relates to the field of medicine, in particular, to pharmacology. The novel dosage form is characterized by the neutral carrier thereof that does not contain chemical, plant-based, or biological compounds - the substances causing side effects and having contraindications to the administration thereof. The dosage form is a neutral carrier with low intensity energy-informational electromagnetic radiation transmitted thereupon from a parent chemical or biological substance, which produces the desired pharmacological effect. The neutral carrier is a liquid (water, aqueous medium), patch, gel, or suspension. The method for the preparation of the dosage form includes transmission of low intensity energy-informational electromagnetic radiation bearing the desired pharmacological effect of the parent chemical, plant-based, or biological substance onto a neutral carrier suitable for interaction with the patient's body. The low intensity energy-informational electromagnetic radiation is transferred onto the neutral carrier from an intermediate carrier, which is suitable for the storage and transmission of low intensity energy-informational electromagnetic signals. The transmission of radiation is previously conducted onto one intermediate carrier; the radiation is wirelessly transmitted through communication media to a second intermediate carrier (optical disc), upon which the neutral carrier is exposed. The neutral carrier exposed on the intermediate carrier is an aqueous medium, such as potable water or saline solution. The result is improved safety, expanded range of the drug use, and elimination of side effects during treatment due to elimination of the direct chemical or pathophysiological effect on the patient's body while using the claimed dosage form, which reproduces main therapeutic properties of the chemical or biological drug

## Description

The claimed group of inventions relates to the field of medicine, in particular to pharmacology, and may be used for the safe, effective, and inexpensive treatment of patients with contraindications to medical preparations in some pharmaceutical forms. The characteristic feature of the claimed novel dosage form of medical compositions is that the neutral carrier thereof does not contain any chemical, plant-based, or biological substances, the moieties causing side effects, and contraindicated for use.

Various solid and liquid dosage forms of pharmaceutical preparations, such as tablets, solutions, infusions, decoctions, tinctures, extractions, potions, musilages, emulsions, suspensions, patches, tampons, soft gelatin capsules, creams, ointments, gels, etc. are approved for medical use.

However, any medication as such can be contraindicated in any known dosage form because of hypersensitivity, which is impossible to predict. Any medication as such can produce side effects and cause individual reactions and side effects, such as rash, itching, redness, severe allergy with facial edema, and pain. Colchicine, for example, is known to cause impaired kidney function, morning bleeding, proximal myo-pathy with elevated creatine phosphokinase levels, and peripheral neuropathy. Additionally, in some cases, small doses of colchicine are known to cause complications resulting in death.

Allopurinol is also known to cause side effects in the form of allergic skin rashes (such as maculopapular rash), dyspepsia, diarrhea, and headaches. Sulfinpyrazone and phenylbutazone cause impaired hematopoiesis and impaired liver function. Patients taking benzomarone report diarrhea and itchy skin rash.

There is a method known in the art for the preparation of dosage forms providing a controlled release of the active ingredient, wherein at least three out of four compressed medications containing an active ingredient or a combination of active ingredients in the tablet composition (compressed medication) that can be varied in the content and the amount thereof are combined; wherein said compressed medications are prepared according to the known procedures by way of combining active ingredients with pharmaceutically acceptable excipients and/or carriers by granulation, tableting and/or coating to yield any pharmaceutically desirable and release profile of the active ingredient or a combination of active ingredients, such as delayed, uniform, or adapted to a certain rhythm (pulsating) release, wherein a compressed medication of the A type comprising four compressed medications, releases no less than 75 % of the active ingredient composition thereof in the course of 45 min.; a compressed medication of the B type releases 100 % of the active ingredient composition thereof no later than in 3 hrs. with the release profile of about 0, and obtained by hydrophilic, lipophilic matrix tablets or a lacquer coating with a controlled diffusion; a composition of the C type with the hydrogen ion activity pH around 6 - 7.5, releases at least 75 % of the active ingredients in the first 45 min., and prepared in a similar manner to the preparation of the coated Composition A tablets, which are resistant to stomach acid; a composition of the D type with the hydrogen ion activity pH around 6 - 7.5, releases 100 % of the active ingredients no later than 3 hrs. with the release profile of about 0, and obtained as matrix tablets, which are resistant to stomach acid, or as a combination of lacquer coatings, which are resistant to stomach acid with a controlled diffusion (RU # 2193396).

There is a method known in the art for the preparation of dosage forms or their precursors by extrusion, wherein said dosage form is a matrix comprising mostly an active ingredient, wherein said matrix comprises a polysaccharide, and/or its derivative, and/or its complex, and/or a saccharide, and/or derivatives thereof as the main forming matrix, and additionally, at least one pharmaceutically effective substance, wherein said matrix is formed by coextrusion with the active ingredient and provides release of the active ingredient (RU # 2208436).

There is a method known in the art for the preparation of dosage forms, wherein an osmotic hydrogel and an osmotically effective solute are combined to yield a composition that expands in volume in the presence of an aqueous liquid; hydroxyalkyl cellulose is dissolved in water to yield a granulation solution; said granulation solution is then sprayed over the composition expanding in volume to yield granules; the pharmaceutical composition, a surface-active ingredient, and the component selected from the group comprising a mono- and a diglyceride are then combined to yield a pharmaceutical composition; said pharmaceutical composition is then encapsulated; said capsule is then filled with the sprayed granular composition; said capsule is then coated with a semipermeable composition to form a membrane permeable to an aqueous liquid; said membrane is perforated to deliver the pharmaceutical composition from said delayed controlled release dosage form over a long period of time (RU # 2227021 prototype).

All known dosage forms by themselves, however, are not free from side effects and adverse reactions to the patient, as essentially all drugs containing chemical or biological substances have some side effects, triggering various pathogenetic effects, which include severe allergic reactions.

Thus, it is advantageous to provide a neutral carrier, preferably water, physiological solution, patch, or any other natural or artificial material, with properties affording (mediating) therapeutic benefits to the body, which is equivalent to the effect of the (parent) pharmaceutical preparation but without a direct use of said composition. In such case, adverse reactions associated with chemical and biological properties of such preparation are eliminated.

Any living organism as a whole and all its systems radiate weak electromagnetic waves (physiological or hormonal) in a wide range of frequencies. Pathogens also have their own frequencies. When diseased, a body creates new wave sources, so called "pathological" or "disharmonious" waves, which disturb the physiological equilibrium. A body becomes indisposed when it can't maintain equilibrium between the physiological and pathological waves. A normal physiological state maintains relative synchronization between various oscillating (wave) processes; while in a pathological state, the oscillating harmony is compromised. This can be expressed as compromised rhythms of the main physiological processes, for example, due to the sharp increase in excitation or inhibition mechanisms occurring in the central nervous system or as altered cortico-subcortical interactions. Severe physical, nervous, psychological, ecological, geopathic, and other stress exacerbate this condition, and at times, themselves become disease sources, while stimulating pathological processes and impaired dynamic equilibrium.

Bioresonance therapy is also known in the art. Bioresonance therapy is a therapy with electromagnetic waves, with which the body structures resonanates. The impact is possible both on the cellular and on the organ level, as well as on a system of organs or the whole body. The main theory behind the resonance use in medicine is that the right frequency and right type of radiation used for treatment can amplify normal (physiological) and suppress pathological oscillations in the human body. The bioresonance effect, therefore, can be directed to neutralize pathological and restore physiological oscillations impaired in pathological states.

The objective of the group of inventions with one joint inventive concept is to provide an effective dosage form that eliminates the need for the direct administration of any chemical or biological substance into a patent's body and a method for the preparation thereof, as well as to expand the arsenal of dosage forms and methods for the preparation thereof.

The technical result of the group of the inventions with one joint inventive concept is improved safety, expanded range of use of the preparations, and elimination of side effects during therapy by removing the direct chemical or pathophysiological effect on the patient's body when using the claimed dosage form, which replicates main therapeutic properties of chemical or biological substances and which is prepared using an intermediate neutral carrier containing and reproducing the typical (of the substance) energy-informational low intensity radiation that had been transmitted thereupon.

The spirit of the invention as to the dosage form is that the dosage form is a neutral carrier with low intensity energy-informational electromagnetic radiation transmitted thereupon from the parent chemical or biological substance, which produces the desired pharmacological effect.

In a preferred embodiment, the neutral carrier is a liquid, patch, gel, suspension, homeopathic pellets, in particular, the liquid is an aqueous medium, such as potable water or saline solution.

The spirit of the invention as to the preparation method of the dosage form is that said method provides transmission of the low intensity energy-informational electromagnetic radiation bearing the desired pharmacological effect of the parent chemical, plant-based, or biological substance onto a neutral carrier suitable for the interaction with the patient's body.

In a preferred embodiment, the low intensity energy-informational electromagnetic radiation is transmitted onto a neutral carrier from an intermediate carrier suitable for the storage and transmission of low intensity energy-informational electromagnetic signals.

In a preferred embodiment, by applying laser radiation or a magnet to the parent chemical or biological substance, the low intensity energy-informational electromagnetic radiation thereof is transmitted onto at least one intermediate carrier where it is stored until transmitted to the neutral carrier.

In some embodiments, transmission of radiation is previously conducted onto one intermediate carrier; the radiation is wirelessly transmitted via communication media to the second intermediate carrier, upon which the neutral carrier is exposed.

In a preferred embodiment, at least one intermediate carrier is an optical disk, wherein information is transmitted and read by a laser or by any other method known in the art.

In some embodiments, transmission of radiation from one intermediate carrier to another is carried out via communication media, such as the Internet, telephone, or power lines.

In some embodiments, the neutral carrier exposed on the intermediate carrier is a liquid, patch, gel, suspension, or homeopathic pellets.

In some embodiments, the neutral carrier exposed on the intermediate carrier is an aqueous medium, such as potable water or saline solution.

The group of inventions is based on the following tenets, which are apparent from and repeatedly confirmed by the modern science:
Informational radiation (field) of organic and inorganic substances, the biological objects with electromagnetic nature, is an oscillating process with characteristically superweak intensity and extremely low frequency. Its characteristics are frequency, wavelength, and amplitude (or spectrum), which are specific to the individual radiation objects (sources). A radiation (field) cannot exist separately from the source or a temporary or a permanent carrier. Radiation (field) can be transmitted by waves or other means from the source onto a carrier. The effect of radiation (field) on a biological object is specific and similar to that of the parent source, which had been transmitted onto a radiation carrier. Such information is harmonious, and a positive regulatory capability thereof towards humans is rather high.

Thus, the task of transmitting specific radiation of pharmaceutical preparations onto solid and liquid neutral carriers, such as water and water medium of living organisms (saline solution), natural or artificial materials, etc. is evidently urgent. Among others, water is an element sensitive to radiation created by chemical and biological preparations, and is most suitable in practical applications as a neutral carrier.

The proposed group of inventions is based on the principle of creating a low intensity electromagnetic background by using a neutral (passive) carrier, which can be water or another substance and materials. The amount and specificity of the electromagnetic properties of said background are determined by the radiation effect of the parent preparation transmitted onto an intermediate hard carrier, such as an optical laser compact disk, which is used for long distance transmissions or transfers.

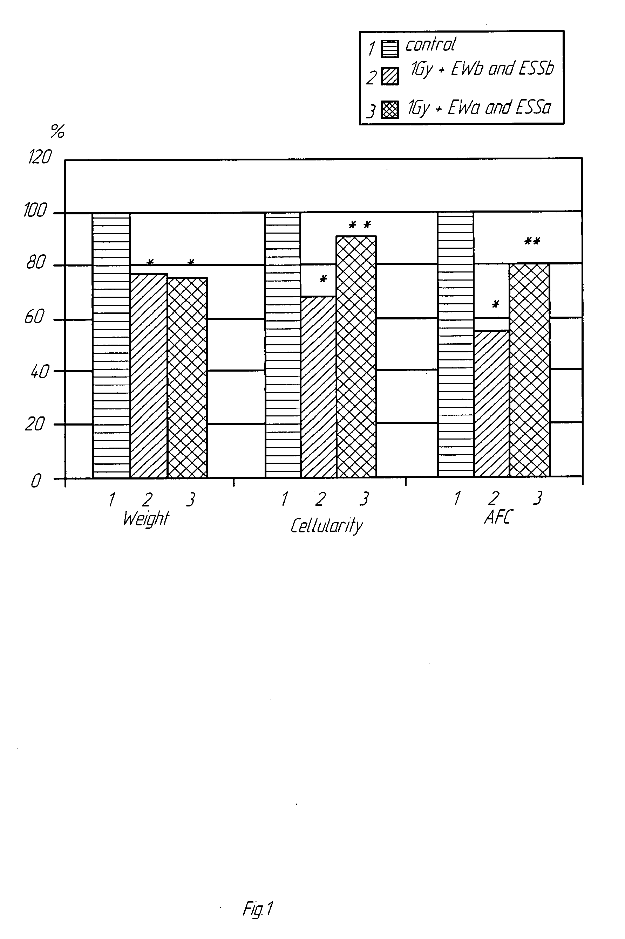

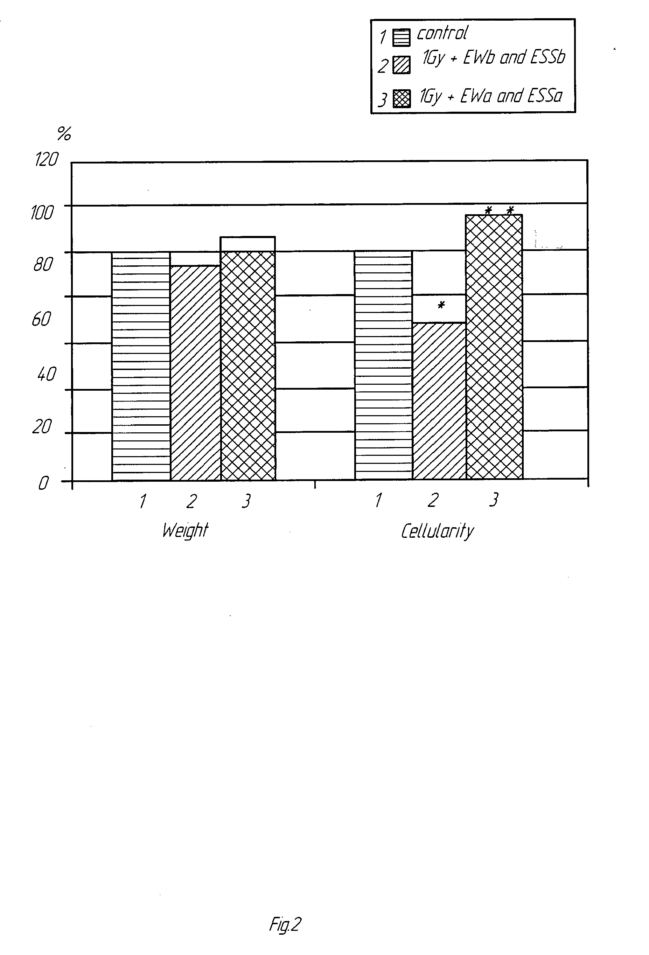

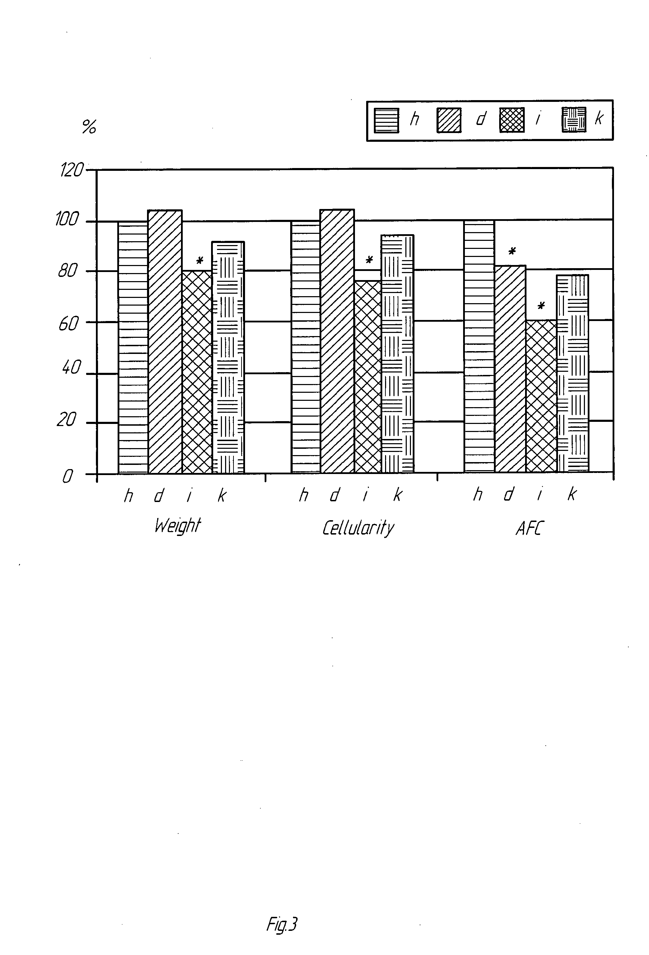

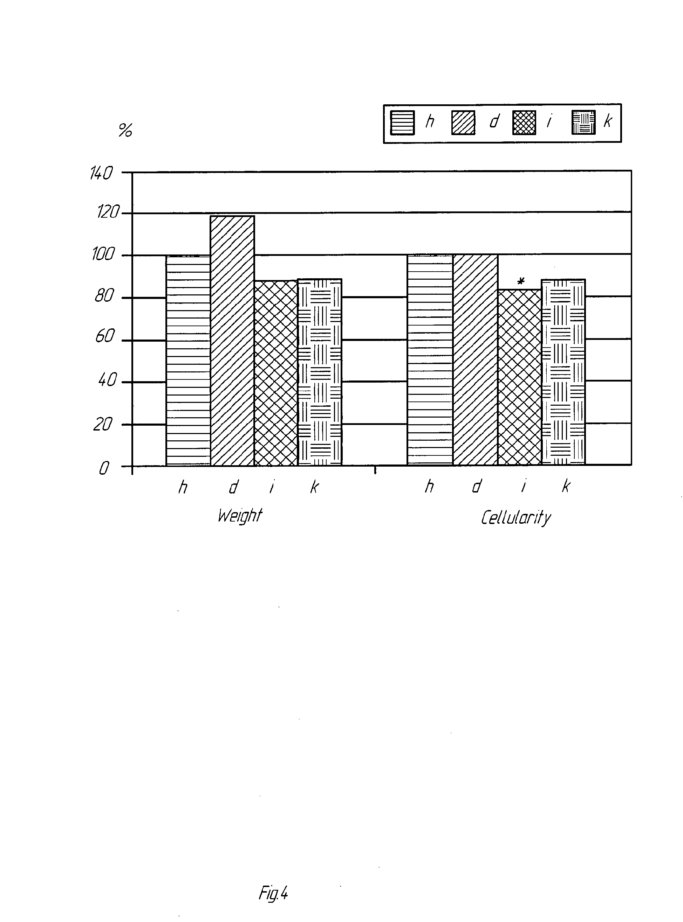

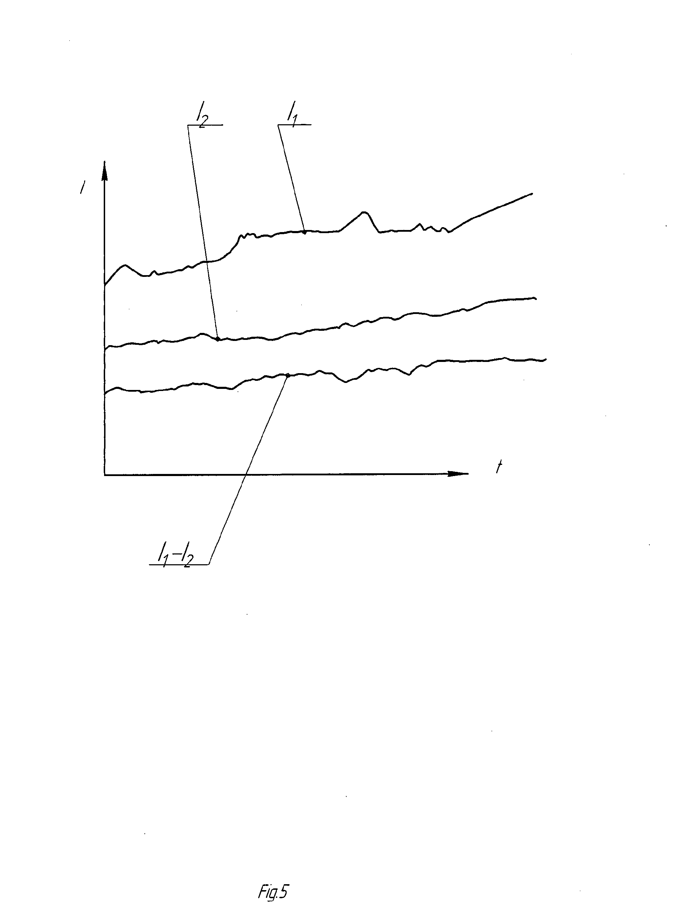

Fig. 1 shows a diagram: weight, cellularity, and antibody-forming cells (AFC) in the murine spleen, whose potable water, in the course of 10 days post ionizing radiation, contained water samples exposed on disks, which received radiation from Arbidol; in the last 8 - 10 days, the mice intraperitoneally received samples of physiological solution (0.5 ml each) exposed on the same disks. Fig. 2 shows a diagram: weight and thymic cellularity in mice, whose potable water, in the course of 10 days post ionizing radiation, contained water samples exposed on disks, which received radiation from Arbidol, in the last 8 - 10 days, the mice intraperitoneally received samples of physiological solution (0.5 ml each) exposed on the same disks. Fig. 3 shows a diagram: weight, cellularity, and antibody-forming cells (AFC) in the murine spleen, whose potable water, in the course of 8 days contained water samples exposed on disks: clean (h), dexamethasone by direct transmission (d), dexamethasone via the Internet (i), and via a contact disk (k). Fig. 4 shows a diagram: weight and thymic cellularity in mice, whose potable water in the course of 8 days contained water samples exposed on disks: clean (h), dexamethasone by direct transmission (d), dexamethasone via the Internet (i), and via a contact disk (k). Fig. 5 shows a diagram of currents flowing between electrodes through the solution placed in the container that was exposed on a solid carrier of the radiation matrix.

A novel dosage form of the Arbidol preparation with immunomodulatory properties is an aqueous medium saturated with radiation from Arbidol as a pure crystalline substance. The aqueous medium is boiled potable water or a saline solution.

The preparation method is as follows:
By applying laser radiation or a magnet to the parent drug substance, the low intensity electromagnetic radiation specific to the (particular) parent sample of the drug substance (pharmaceutical preparation) is transferred in the form of energy-informational signals to the first intermediate carrier (mostly, a CD or a DVD, or another device suitable for the energy-informational signal reception). Transmission of the converted signal to the first intermediate carrier is conducted by modulation/demodulation of radiation from the standard transmitting equipment by energy-informational signals. As the first intermediate carrier enters the transmitting equipment, it triggers transmission of the electromagnetic wave signal over the wire and wireless networks (lines), which include Internet, to the receiver's entry point, followed by the signal transmission to the receiving side and then transmission of said signal to the second intermediate carrier (analogous or similar to the first).

The use of the second intermediate carrier for the formation of the novel dosage form is conducted by exposing ordinary potable, mineral or sea water, biological fluid (serum, blood, or blood substitute), patch, gel, or some natural or artificial materials on said dosage form at room temperature and natural light during a short time period. As a result, an energy-informational signal is induced (reproduced) on said objects as low intensity electromagnetic radiation specific to (characteristic of) the parent drug substance - the source of radiation. Thus, said objects acquire the form of the novel dosage form - the carrier and the source of the energy-informational signal, as low intensity electromagnetic radiation, which is identical to the parent chemical or biological pharmaceutical preparation, and can be used accordingly, when indicated, as a novel dosage form, the method of the preparation and use thereof.

For instance, a parent preparation, such as Arbidol or Diclofenac as a crystalline substance is used for laser transmission onto a solid carrier, which can be called "first intermediate carrier", said radiation is then transmitted over a network to the "second intermediate carrier", and from there, to the aqueous medium, which is exposed for several minutes in a container on an operational carrier.

Radiation transfer can be done directly onto the last operational carrier, or can be first transferred to one intermediate carrier, from where the transferred thereupon (recorded) information is transmitted by electrical connection, e.g., over the Internet, and then wirelessly transmitted (recorded) onto the second intermediate carrier, with an aqueous medium exposed thereupon in a container. Radiation on the disk affects the exposed water altering its supramolecular structure by triggering formation of coherent domains n the water, which is confirmed experimentally by the reduced electronic work function and increased water (H₂O) vaporization rate. Consequently, the electromagnetic signal from the neutral carrier radiation senses the DNA cells.

The intermediate carrier is, preferably, a laser compact disk (CD). The intermediate temporary carrier can be a CD, or a DVD, or other devices suitable for radiation reception and storage and convenient for use.

Radiation transmission can be conducted, for example, by the method cited in the WO 201162518 information source, or by another method.

The study included a total of 86 radiations from chemical and plant-based preparations (herbal mix). The device according to the present invention was administered to 45 subjects in Russia, 30 subjects in Ukraine, and 8 subjects in Japan. In total, children under 3 years of age - 9; children 3 - 14 years of age - 5; the rest (39 subjects) were adults with various medical conditions. Most complained of acute disease symptoms. All the subjects reported a fast reduction of symptoms as compared to the previous course of the disease.

Industrial applicability of the claimed device and method is supported by the practical application results presented below as protocols comparing the device obtained by the claimed method while using Arbidol with the device obtained in a similar way but using Dexamethasone, a preparation with an opposite action. Industrial applicability of the claimed device and method is also supported by the practical application results presented below as the results of a clinical follow-up of the patients receiving radiation from the device prepared by the claimed method while using Diclofenac.

### PROTOCOL

### of the study assessing immunomodulatory properties of water and saline solution exposed on compact disks containing radiation reflected from Arbidol and Dexamethasone

**Study objective:** to determine the absence or presence of immunomodulatory properties of saline solution (SS) or boiled water (W) exposed on compact disks containing transmitted (reflected) radiation from the antiviral immunostimulant Arbidol and from Dexamethasone (Dexone), an analog to the glucocorticoid hormone, known to exhibit immunosuppressive activity.

The studies were conducted in accordance with the requirements for the evaluation of immunotrophic activity of pharmaceutical preparations set forth in the Handbook for Experimental (Nonclinical) Study of Novel Pharmaceutical Preparations. M. 2000. Thymic and splenic weights and cellularity (number of live nucleus-containing cells in the homogenate) and the amount of antibody-forming cells (AFC) in the spleen, formed as a result of the immune response to the ram's red blood cells, were chosen as indicators of the condition of the immune system organs.

### Study of the Arbitol Radiation Properties

The parent preparation was the pure crystalline drug Arbidol prepared by a patented procedure.

The object of the study was immunomodulatory activity of boiled water and saline solution (pharmacy drug, hereinafter "saline") exposed on a clean laser compact disk or a working carrier (laser compact disc containing transmitted informational radiation from Arbidol).

Testing the effect of the pharmaceutical preparation Arbidol on water.

Electrochemical cells (containers with water and electrodes) were placed on the surface of the radiation carrier (disk) from the pharmaceutical preparation Arbidol, and the currents in the cells during exposition were continuously recorded.

The upper curve on Fig. 5 corresponds to current I₁ in the cell set on top of the disk containing radiation from Arbidol. The middle curve shows the dynamics of current I₂ in the cell set on top of a clean disk (no radiation from Arbidol).

The lower curve 3 is the difference between currents I₁ and I₂.

The curves indicate that the currents' dynamics are quite different. Because the curves reflect changes in the state of water molecules, in particular, changes in the share of free water molecules that are responsible for the electronic work function, the curves may be used to evaluate the state of water, in particular, the degree of excitation of the molecules that was triggered by radiation from the intermediate (temporary) carrier (disk).

This confirms that such intermediate carrier, a laser compact disk, is indeed a carrier of the radiation transmitted thereupon, whereupon said radiation can be used to affect water or saline solution to yield the claimed device. This study demonstrated that the effect of the device prepared by the claimed method is precisely that of Arbidol. In other instances, sea or mineral water, both natural and artificial, can be used as such solutions.

The tests were conducted on male laboratory F₁ hybrid mice (CBA x B₆) weighing 22 - 24 g, held in a vivarium on a standard diet. A group of intact mice, not exposed to anything, served as the control. Two groups of laboratory mice were completely exposed to ionizing radiation on the ray -1 device at the 1 Gy sublethal (not causing death) dose. One of the groups, for 10 days, had unlimited access to drinking boiled water exposed on a clean disk (EWb), the other group received water that had been exposed for 24 hrs. on working carriers - compact disks with Arbidol radiation transmitted thereupon (EWa). In the last 8 - 10 days, the mice in both groups were intraperitoneally administered 0.5 ml saline solution exposed for 24 hrs. on a clean disk (ESSb) or saline solution exposed on the disk with reflected Arbidol radiation (ESSa). Then the mice in all groups were intraperitoneally immunized with ram's red blood cells at 1x 10⁸; in 4 days, the mice were euthanized by decapitation under ether anesthesia, the spleen and thymus were extracted, homogenized in medium 199; the stroma was isolated on a capron filter, and the amount of nucleus-containing cells as well as the number of antibody-forming cells (AFC) were determined by Cunning-ham's technique.

The experiment yielded the following results (Table 1, Fig. 1 and 2): the weight of the spleen, cellularity thereof, and the AFC content therein as well as the thymic cellularity in the group of mice receiving ionized radiation at the sublethal 1 Gy dose and later receiving EWb and ESSb exposed on clean disks, were lower than those of the control group of intact mice. Said results reflect the impact of ionized radiation as disorders of highly sensitive organs of the immune system. The group of mice (1 Gy + EWa and ESSa) receiving water and saline exposed on disks with radiation reflected from Arbidol (EWa and ESSa, respectively) demonstrated elevated thymic and splenic cellularity as well as an elevated AFC content in the spleen (important functional indicator) practically at the control level in comparison to the group of radiated mice receiving EWb and ESSb. Thus, both water and saline, upon exposition on a disk with Arbidol radiation become capable of restoring the immune system indicators impaired by radiation.

Immunological indicators (M±m) of the control group and mice irradiated at the 1 Gy dose, whose drinking bowls, right after irradiation thereof at the 1 Gy dose, were filled (for 10 days) with samples of water exposed on disks (EWb and ESSb) and who were also intraperitoneally administered 0.5 ml each of saline exposed onto disks (ESSb and ESSa) daily, 8 - 10 days post irradiation, are shown in Table 1.

**Table 1**

| Group of Mice | Spleen | | | Thymus | |
|---|---|---|---|---|---|
| | Weight, mg | Cellularity, 1 x 10⁶ | AFC Content, 1 x 10³ | Weight, mg | Cellularity, 1 x 10⁶ |
| Control | 133 ± 7.8 (100 ± 5.9) | 185 ± 10.6 (100 ± 5.7) | 221 ± 21.3 (100 ± 9.6) | 43.7 ± 4.5 (100 ±10.3) | 67.2 ± 4.2 (100 ± 6.3) |
| 1 Gy + EWb and ESSb | 103 ± 2.7* (77.4 ± 2.0) | 127 ± 6.5* (68.6 ± 3.5) | 126 ± 6.4* (57.0 ± 2.9) | 40.6 ± 1.9 (92.9 ± 4.3) | 45.3 ± 3.4* 67.4 ± 5.1 |
| 1 Gy + EWa and ESSa | 101 ± 4.2* (75.9 ± 3.2) | 170 ±13.1** (92.0 ± 7.1) | 182 ±18.7** (82.4 ± 8.4) | 46.1 ± 3.2* (105 ± 7.3) | 76.7 ± 4.7** (114 ± 7.0) |

| | | | | | |
|---|---|---|---|---|---|
| *Note*: significant (p<0,05) difference: * from the control; ** - from group 1 Gy + Wb + ESSb. In parentheses - % to the control. | | | | | |

### Studying the properties of Dexamethasone (Dexone) radiation.

As data from Table 2 and Fig. 3 and 4 show, the claimed water samples used by laboratory mice with free access to water reduce immune reactivity. The group of mice receiving water exposed on the disk with dexamethasone radiation directly transmitted thereupon (reflected) showed a decreased humoral immune response, as demonstrated by the reduced amount of antibody-forming cells (AFC) in the spleen. The group of mice, receiving water for 8 days, which had been exposed for 24 hrs. on the disk containing reflected Dexamethasone radiation that had been transmitted via the Internet, demonstrated reduced splenic weight, cellularity, and AFC content as well as reduced thymic cellularity.

Thus, upon exposition on the disk containing radiation from Dexamethasone, water can reduce the immune reactivity indicators in the intact mice. Hence, Dexamethasone's emission replicates the properties thereof in water.

Immunological indicators (M±m) of mice whose drinking bowls were filled for 8 days with water samples exposed on different disks, are shown in Table 2.

**Table 2**

| Group of Mice | Spleen | | | Thymus | |
|---|---|---|---|---|---|
| | Weight, mg | Cellularity, 1 x 10⁶ | AFC Content, 1 x 10³ | Weight, mg | Cellularity, 1 x 10⁶ |
| Water from clean disk ***h*** (control) | 139 ± 6.0 (100 ± 4.3) | 228 ± 8.6 (100 ± 3.8) | 284 ± 14.3 (100 ± 5.0) | 46.2 ± 4.2 (100 ±9.1) | 97.3 ± 4.7 (100 ± 4.8) |
| Water from disk ***d*** (Dexamethasone directly) | 143 ± 10.8 (100.3±7.8) | 225 ± 6.2 (103 ± 2.8) | 234±15.3* (82.4 ±5.4) | 54.3 ± 3.4 (118±7.4) | 96.0 ± 6.3 99.0 ± 6.5 |
| Water from disk ***i*** (Dexamethasone via Internet) | 113 ± 5.1* (81.3 ± 3.7) | 168 ±11.0* (77.1 ±5.0) | 174 ±27.8* (61.3 ±9.8) | 40.5 ± 2.9 (87.7±6.3) | 80.5 ± 3.1* (82.7 ±3.2) |
| Water from disk ***k*** (contact) | 128 ± 6.4 (92.0 ± 4.6) | 207 ± 13.0 (95.0 ±6.0) | 224 ± 52 (78.9±18.3) | 40.7 ± 3.3 (88.1±7.1) | 84.7 ± 5.7 (87.0 ±5.9) |

| | | | | | |
|---|---|---|---|---|---|
| *Note*: * - significant (p<0,05) difference from the group receiving water from clean disk **h**. In parentheses - % to the control. | | | | | |

A similar experiment was conducted over 5 days with the intraperitoneal of saline exposed on disks containing radiation reflected from Dexamethasone. There were no differences from the control. The absence of the effect is presumed to stem from the decreased disk activity associated with the prolonged storage thereof, since the saline was exposed 30 days after irradiation. Thus, there is no table reflecting the specific data.

The results are clearly confirmed by the diagrams shown on Fig. 1 - 4.
Fig. 1, 2 indicate: * - p<0,05 to the control group; ** - p<0,05 to the group 1 Gy + Wb and ESSb. X-axis - indicator %, y-axis -indicators under study. Fig. 1 - weight, cellularity, and antibody-forming cell content (AFC) in the spleen of mice whose drinking bowls, after irradiation, were filled for 10 days with samples of water exposed on disks, and who were intraperitoneally administered 0.5 ml each of saline exposed on the same disks.
Fig. 2 - weight and cellularity in the thymus of mice whose drinking bowls were filled for 10 days after irradiation with samples of water exposed on disks, and who, in the last 8-10 days, were intraperitoneally administered 0.5 ml each of saline samples exposed on the same disks.
Fig. 3,4 indicates: * - p<0,05 to group **h**
   X-axis - indicator %, y-axes - indicators under study
Fig. 3 - weight, cellularity, and antibody-forming cell content (AFC) in the spleen of mice whose drinking bowls were filled for 8 days with samples of water exposed on disks: clean (h), Dexamethasone direct (d), Dexamethasone via Internet (i), and contact disk (k).
Fig. 4 - weight and cellularity in the thymus of mice whose drinking bowls were filled for 8 days with samples of water exposed on disks: clean (h), Dexamethasone direct (d), Dexamethasone via Internet (i), and contact disk (k).

The pharmaceutical product prepared per the claimed method was tested clinically. Patient S.Zh.A., age 47, height 156 mm, weight 52 kg, BP 105/60 mm Hg, pulse 78, complaining of pain in the spine and pelvis. **Diagnosis:** cancer of the left breast, metastases to the spine and pelvis (CT-scan data). Examined 3 days post CT-scan.

### Testing and Follow-up

| Indicator | Original condition | Radiation | Post 2 weeks | Post 1 Month |
|---|---|---|---|---|
| State | 2/2 | 3/4 | 3/1 | 3/4 |
| CT | 9 | 18 | 16 | 20 |
| AR | low 4 | av. 3 | av. 3 | av.4 |
| PI | 16 | 7 | 8 | 7 |
| Left breast | 6 | 14 | 12 | 16 |
| Rad. stress | 4 | 1 | n/a | n/a |
| Immune syst. | Extreme Exhaustion | Stress | Stress | Stress |

**Conclusion**: biofunctional indicators in total and in particular were improved and radioactive stress completely disappeared.

### Conclusion.

The experimental results lead to a valid conclusion that the device, which is an aqueous medium wherein radiation from the parent chemical drug Arbidol as a pure crystalline substance was transmitted thereupon, exhibits immunomodulatory activity; transmission of radiation of the specific pharmaceutical preparations Arbidol and Dexamethasone, with opposite immunomodulatory properties, onto compact discs enables them to induce water or saline solutions with the activity similar to that of the parent preparations.

Said results indicate the applicability and potential use of the claimed device as an immunomodulatory agent to reduce the effects of ionizing radiation, in particular, during radiation therapy used for the treatment of cancer patients or for the victims of radiation leaks; said results also validly indicate that the claimed device, as an immunomodulatory agent same as Arbidol, exhibits antiviral activity.

### PROTOCOL

### of the study assessing the effect of water exposed on compact disks containing radiation reflected from Diclofenac

A disk with radiation transmitted from Diclofenac was placed in the area with natural light (on the window sill, in our case); a glass filled with drinking water (non-carbonated) was placed on top of said disk. In 30 - 40 min., the water is ready for consumption. During the day, the patients were drinking said water when thirsty. As the water was used up, the glass was refilled. Irradiated water consumption does not have any diet restrictions. The patients' diet was complete in content and variety, with no excesses.

The Vegetative Resonance Test (VGT) was used to control results. VRT indicators are most frequently used for testing patients receiving water irradiated with medications.
- Overall condition. A quantitative examination of the overall systemic condition, organs, and the system. The scale is from 1 to 7. Each paragraph is subdivided into several subparagraphs. The ideal state is 7/4.
- CT - connective tissue (matrix of the human body). Present in all organs and systems. The overall health and that of the organs and systems are expressed through the intensity of the connective tissue oscillations. The scale is from 0 to 100. The ideal state is 100.

- Adaptation reserves (AR). Body's potential. Waning (5 deg.), low (4), average (4), good (5), high (6), very high (5). 29 gradations overall. Ideal indicator - very high adaptation reserves - 5 deg.
- Photon Index - information exchange between cells, which reflects the status of the body as a self-regulating system. The scale is from 1 to 22, ideal indicator - 1.

The questionnaires and actual treatment results for specific patients are shown in tables below.

### Clinical observation of Patient G. receiving Diclofenac radiation

**Table 1. Patient's questionnaire. Diagnosis: GERD. Reactive arthritis, act 1, NF2**

| | |
|---|---|
| Test date | 11/10/2011 |
| BP, mm Hg | 130/80 |
| Pulse, times/min | 76 |
| Blood sugar, mmol/L | 4.6 |
| Patient's complaints | Dull joint pain: ankle, wrist, knee. Acidic taste in the mouth, soft stool. |
| Blood chemistry | Hb 130, L - 7.4, Sed. Rate 22 |
| Urine test | Sp. gravity 1020. Protein abs, L 2 - 3 per HPF |
| Conclusion on the effect of the radiation on patient | Positive clinical effect and dynamics of integrative indicators |

**Table 2. VRT Indicators**

| Paragraph numbers | Indicators | Original condition | Condition with radiation | Patient's condition w/o radiation 1 month later |
|---|---|---|---|---|
| 1 | Overall condition | 6 | 8 | 8 |
| 2 | CT | 54 | 78 | 70 |
| 3 | IR | average | good | good |
| 4 | PI | 16 | 15 | 15 |
| 5 | Joints D6/CT | 44 | 68 | 66 |

### Clinical observation of Patient L. receiving radiation - Diclofenac

**Table 1. Patient's questionnaire. Diagnosis: Reactive arthritis, act 1, NF2**

| | |
|---|---|
| Test date | 11/20/2011 |
| BP, mm Hg | 120/80 |
| Pulse, times/min | 78 |
| Blood sugar, mmol/L | 4.8 |
| Patient's complaints | Joint pain: ankle, knee, heel, back, low-grade fever |
| Blood chemistry | Hb 130, L - 11, Sed. Rate 33 |
| Urine test | Ureaplasma present, L - 10 - 15, b-0.033 |
| Conclusion on the effect of the radiation on patient | With radiation, pain diminished, integrative indicators improved |

**Table 2. VRT Indicators**

| Paragraph numbers | Indicators | Original condition | Condition with radiation | Patient's condition w/o radiation 1 month later |
|---|---|---|---|---|
| 1 | Overall condition | 5 | 6 | 6 |
| 2 | CT | 34 | 70 | 64 |
| 3 | IR | average | good | good |
| 4 | PI | 15 | 15 | 15 |
| 5 | Joints D6/CT | 33 | 68 | 60 |

### Clinical observation of Patient K. receiving radiation - Diclofenac

**Table 1. Patient's questionnaire. Bekhterev's disease, degenerative joint disease**

| | |
|---|---|
| Test date | 11/3/2011 |
| BP, mm Hg | 130/80 |
| Pulse, times/min | 76 |
| Blood sugar, mmol/L | 5.1 |
| Patient's complaints | Back pain from neck to coccyx |
| Blood chemistry | Hb 97, L - 10.3, Sed. Rate 47 |
| Urine test | Sp. Gravity 1010, acidity, L - 5-7 |
| Conclusion on the effect of the radiation on patient | No clinical improvement, insignificant dynamics of integrative indicators |

**Table 2. VRT Indicators**

| Paragraph numbers | Indicators | Original condition | Condition with radiation | Patient's condition w/o radiation 1 month later |
|---|---|---|---|---|
| 1 | Overall condition | 4 | 5 | 4 |
| 2 | CT | 46 | 50 | 46 |
| 3 | IR | average | average | average |
| 4 | PI | 15 | 15 | 15 |
| 5 | Joints D6/CT | 36 | 38 | 36 |

### Clinical observation of Patient R. receiving radiation - Diclofenac

**Table 1. Patient's questionnaire. Diagnosis: Rheumatoid arthritis, polyarthritis act 1, st. 2, NF2**

| | |
|---|---|
| Test date | 11/3/2011 |
| Height, sm | 170 |
| BP, mm Hg | 160/90 |
| Pulse, times/min | 80 |
| Blood sugar, mmol/L | 5.8 |
| Patient's complaints | Joint pain: wrist, foot, right knee, elevated BP |
| Blood chemistry | Hb 130, L - 10, Sed. Rate 33 |
| Urine test | Sp. Gravity 1010, acidity, L - 2 - 4 |
| Conclusion on the effect of the radiation on patient | Joint pain diminished, positive dynamics of integrative indicators, but short term liver pain |

**Table 2. VRT Indicators**

| Paragraph numbers | Indicators | Original condition | Condition with radiation | Patient's condition w/o radiation 1 month later |
|---|---|---|---|---|
| 1 | Overall condition | 4 | 6 | 4 |
| 2 | CT | 45 | 60 | 50 |
| 3 | IR | average | good | good |
| 4 | PI | 18 | 17 | 18 |
| 5 | Joints D6/CT | 46 | 50 | 46 |

### Clinical observation of Patient K. receiving radiation - Diclofenac

**Table 1. Patient's questionnaire. Degenerative Joint Disease with knee joints mostly affected**

| | |
|---|---|
| Test date | |
| BP, mm Hg | 150/80 |
| Pulse, times/min | 78 |
| Blood sugar, mmol/L | 6.2 |
| Patient's complaints | Knee joint pain |
| Blood chemistry | Normal |
| Urine test | |
| Conclusion on the effect of the radiation on patient | Diminished pain, improved integrative indicators |

**Table 2. VRT Indicators**

| Paragraph numbers | Indicators | Original condition | Condition with radiation | Patient's condition w/o radiation 1 month later |
|---|---|---|---|---|
| 1 | Overall condition | 62 | 78 | 70 |
| 2 | CT | 60 | 77 | 70 |
| 3 | IR | average | good | good |
| 4 | PI | 15 | 14 | 14 |
| 5 | Joints D6/CT | 46 | 60 | 58 |

### Clinical observation of Patient R. receiving radiation - Diclofenac

**Table 1. Patient's questionnaire. Reactive arthritis, chronic colitis**

| | |
|---|---|
| Test date | 10/20/2011 |
| BP, mm Hg | 130/80 |
| Pulse, times/min | 76 |
| Blood sugar, mmol/L | 4.8 |
| Patient's complaints | Wrist joint pain |
| Blood chemistry | Normal |
| Urine test | |
| Conclusion on the effect of the radiation on patient | Joint pain stopped with radiation |

**Table 2. VRT Indicators**

| Paragraph numbers | Indicators | Original condition | Condition with radiation | Patient's condition w/o radiation 1 month later |
|---|---|---|---|---|
| 1 | Overall condition | 62 | 70 | 70 |
| 2 | CT | | | |
| 3 | IR | good | high | high |
| 4 | PI | 15 | 14 | 14 |
| 5 | Joints D6/CT | 52 | 74 | 68 |

### Clinical observation of Patient N. receiving radiation - Diclofenac

**Table 1. Patient's questionnaire. Gout, gouty arthropathy, NF2**

| | |
|---|---|
| Test date | 11/09/2011 |
| BP, mm Hg | 130/80 |
| Pulse, times/min | 76 |
| Blood sugar, mmol/L | 5.2 |
| Patient's complaints | Knee joint pain, metatarsal pain |
| Blood chemistry | Hb 130, L - 10, Sed. Rate 22 |
| Urine test | Sp. Gravity 1010, L - 5-6 |
| Conclusion on the effect of the radiation on patient | Clinical effect is weakly pronounced |

**Table 2. VRT Indicators**

| Paragraph numbers | Indicators | Original condition | Condition with radiation | Patient's condition w/o radiation 1 month later |
|---|---|---|---|---|
| 1 | Overall condition | 53 | 60 | 54 |
| 2 | CT | | | |
| 3 | IR | average | good | average |
| 4 | PI | 16 | 16 | 16 |
| 5 | Joints D6/CT | 52 | 58 | 54 |

### Clinical observation of Patient T. receiving radiation - Diclofenac

**Table 1. Patient's questionnaire. Ankylosing spondylitis, juvenile arthritis**

| | |
|---|---|
| Test date | 11/20/2011 |
| BP, mm Hg | 120/80 |
| Pulse, times/min | 76 |
| Blood sugar, mmol/L | 4.8 |
| Patient's complaints | Pain in the spine, right knee joint |
| Blood chemistry | Hb 138, L - 5.6, Sed. Rate 15 |
| Urine test | Sp. Gravity 1010, acidity, L - 1- 2 |
| Conclusion on the effect of the radiation on patient | Diminished joint pain, improved integrative indicators |

**Table 2. VRT Indicators**

| Paragraph numbers | Indicators | Original condition | Condition with radiation | Patient's condition w/o radiation 1 month later |
|---|---|---|---|---|
| 1 | Overall condition | 5 | 7 | 7 |
| 2 | CT | 35 | 67 | 60 |
| 3 | IR | average | good | good |
| 4 | PI | 15 | 15 | 15 |
| 5 | Joints D6/CT | 20 | 50 | 50 |

### Clinical observation of Patient K. receiving radiation - Diclofenac

**Table 1. Patient's questionnaire. Rheumatoid arthritis act 1, st. 2, NF2, degenerative joint disease, diabetes mellitus type 2**

| | |
|---|---|
| Test date | 11/22/2011 |
| BP, mm Hg | 140/80 |
| Pulse, times/min | 80 |
| Blood sugar, mmol/L | 5.6 |
| Patient's complaints | Joint pain: wrist, foot, knees, overall lack of energy |
| Blood chemistry | Hb 137, L - 7.2 Sed. Rate 22 |
| Urine test | Sp. Gravity 1015, weakly alkaline, L - 1-3, glucose - 5 |
| Conclusion on the effect of the radiation on patient | Insignificant pain alleviation, weak dynamics of indicators |

**Table 2. VRT Indicators**

| Paragraph numbers | Indicators | Original condition | Condition with radiation | Patient's condition w/o radiation 1 month later |
|---|---|---|---|---|
| 1 | Overall condition | 4 | 5 | 4 |
| 2 | CT | 31 | 40 | 33 |
| 3 | IR | average | average | average |
| 4 | PI | 17 | 16 | 17 |
| 5 | Joints D6/CT | 20 | 25 | 24 |

### Clinical observation of Patient S. receiving radiation - Diclofenac

**Table 1. Patient's questionnaire. Scleroderma, Reynaud's syndrome, hypothyroidism**

| | |
|---|---|
| Test date | 11/18/2011 |
| BP, mm Hg | 120/70 |
| Pulse, times/min | 66 |
| Blood sugar, mmol/L | 4.7 |
| Patient's complaints | Hair loss, chills, constipation, sclerodactylia, cold extremities, transient joint pain |
| Blood chemistry | Hb 120, L - 7.8 Sed. Rate 11 |
| Urine test | Sp. Gravity 1010, weak acidity, L - 1-2, |
| Conclusion on the effect of the radiation on patient | Joint pain alleviated, positive dynamics of integrative indicators |

**Table 2. VRT Indicators**

| Paragraph numbers | Indicators | Original condition | Condition with radiation | Patient's condition w/o radiation 1 month later |
|---|---|---|---|---|
| 1 | Overall condition | 50 | 60 | 55 |
| 2 | CT | 36 | 45 | 40 |
| 3 | IR | low | average | average |
| 4 | PI | 16 | 16 | 16 |
| 5 | Joints D6/CT | 20 | 32 | 32 |

### Clinical observation of Patient R. receiving radiation - Diclofenac

**Table 1. Patient's questionnaire. Rheumatoid arthritis, polyarthritis with visceral manifestations: anemia, lymphadenopathy act 3, st. 3, NF2**

| | |
|---|---|
| Test date | 11/14/2011 |
| BP, mm Hg | 140/80 |
| Pulse, times/min | 78 |
| Blood sugar, mmol/L | 5.5 |
| Patient's complaints | Pain in wrist and foot joints |
| Blood chemistry | Hb 103, L - 11.1 Sed. Rate 51 |
| Urine test | Sp. Gravity 1010, alkaline, L - 3-5 |
| Conclusion on the effect of the radiation on patient | Pain is not alleviated, weak dynamics of indicators |

**Table 2. VRT Indicators**

| Paragraph numbers | Indicators | Original condition | Condition with radiation | Patient's condition w/o radiation 1 month later |
|---|---|---|---|---|
| 1 | Overall condition | 40 | 45 | 40 |
| 2 | CT | 32 | 38 | 33 |
| 3 | IR | low | average | low |
| 4 | PI | 17 | 17 | 17 |
| 5 | Joints D6/CT | 20 | 26 | 22 |

### Clinical observation of Patient D. receiving radiation - Diclofenac

**Table 1. Patient's questionnaire. Classic psoriasis, psoriatic arthropathy,**

| | |
|---|---|
| Test date | 11/14/2011 |
| BP, mm Hg | 160/80 |
| Pulse, times/min | 76 |
| Blood sugar, mmol/L | 6.1 |
| Patient's complaints | Pain in wrist and foot joints, psoriatic plaques, elevated BP |
| Blood chemistry | Hb 114, L - 6.1 Sed. Rate 38 |
| Urine test | Sp. Gravity 1014, acidic, L - 1-2 |
| Conclusion on the effect of the radiation on patient | Positive dynamics of pain syndrome and integrative indicators |

**Table 2. VRT Indicators**

| Paragraph numbers | Indicators | Original condition | Condition with radiation | Patient's condition w/o radiation 1 month later |
|---|---|---|---|---|
| 1 | Overall condition | 50 | 60 | 60 |
| 2 | CT | 48 | 58 | 52 |
| 3 | IR | average | average | good |
| 4 | PI | 17 | 16 | 17 |
| 5 | Joints D6/CT | 22 | 42 | 40 |

### Clinical observation of Patient S. receiving radiation - Diclofenac

**Table 1. Patient's questionnaire. New-onset of rheumatoid arthritis, act 1, st. 1, NF2**

| | |
|---|---|
| Test date | 11/24/2011 |
| BP, mm Hg | 120/70 |
| Pulse, times/min | 78 |
| Blood sugar, mmol/L | 4.3 |
| Patient's complaints | Pain in wrist joints, irritability, lack of energy |
| Blood chemistry | Hb 128, L - 7.5 Sed. Rate 20 |
| Urine test | Sp. Gravity 1020, acidic, L - 1-2 |
| Conclusion on the effect of the radiation on patient | Pain in wrist joints alleviated, positive dynamics of indicators |

**Table 2. VRT Indicators**

| Paragraph numbers | Indicators | Original condition | Condition with radiation | Patient's condition w/o radiation 1 month later |
|---|---|---|---|---|
| 1 | Overall condition | 55 | 70 | 65 |
| 2 | CT | 55 | 77 | 68 |
| 3 | IR | waning | average | average |
| 4 | PI | 15 | 15 | 15 |
| 5 | Joints D6/CT | 40 | 60 | 60 |

### Clinical observation of Patient L. receiving radiation - Diclofenac

**Table 1. Patient's questionnaire. Ankylosing spondylitis act 1, st. 4, NF2**

| | |
|---|---|
| Test date | 11/10/2011 |
| BP, mm Hg | 130/80 |
| Pulse, times/min | 78 |
| Blood sugar, mmol/L | 5.1 |
| Patient's complaints | Back and ankle joint pain |
| Blood chemistry | Hb 137, L - 20 Sed. Rate 26 |
| Urine test | Sp. Gravity 1010, weakly acidic, L - 1-2 |
| Conclusion on the effect of the radiation on patient | Joint pain alleviated, positive dynamics of integrative indicators |

**Table 2. VRT Indicators**

| Paragraph numbers | Indicators | Original condition | Condition with radiation | Patient's condition w/o radiation 1 month later |
|---|---|---|---|---|
| 1 | Overall condition | 45 | 60 | 60 |
| 2 | CT | 34 | 55 | 50 |
| 3 | IR | average | good | good |
| 4 | PI | 15 | 15 | 15 |
| 5 | Joints D6/CT | 20 | 40 | 36 |

### Clinical observation of Patient P. receiving radiation - Diclofenac

**Table 1. Patient's questionnaire. Gout, gouty arthropathy, hypertension, st. 2, r3**

| | |
|---|---|
| Test date | 11/10/2011 |
| BP, mm Hg | 130/80 |
| Pulse, times/min | 78 |
| Blood sugar, mmol/L | 5.1 |
| Patient's complaints | Elbow and knee joint pain |
| Blood chemistry | Hb 121, L - 8.7 Sed. Rate 25 |
| Urine test | |
| Conclusion on the effect of the radiation on patient | Positive effect on pain syndrome and integrative indicators |

**Table 2. VRT Indicators**

| Paragraph numbers | Indicators | Original condition | Condition with radiation | Patient's condition w/o radiation 1 month later |
|---|---|---|---|---|
| 1 | Overall condition | 40 | 55 | 50 |
| 2 | CT | 36 | 56 | 52 |
| 3 | IR | average | average | average |
| 4 | PI | 15 | 15 | 15 |
| 5 | Joints D6/CT | 20 | 40 | 40 |

### PROTOCOL

### of the study assessing the effect of water exposed on compact disks containing radiation reflected from Samprost

Patient V.P.L., 60 yeas old, 107 kg weight, 187 cm height, BP 145/95 mm Hg, 62 pulse. Frequent urination, perineum and groin pain. **Diagnosis**: chronic prostatitis, acute. **Ultrasound**: enlarged prostate, diffuse hyperechogenicity. **Test:** prostatic secretion: pH 7.7, WBC 30 per high power field, RBC - few per HPF.

### Testing and Case Follow-up

| Indicator | Original condition | Radiation | In 1 month | In 2 months |
|---|---|---|---|---|
| Condition | 3/2 | 5/1 | 4/4 | 4/2 |
| CT | 34 | 56 | 48 | 48 |
| IR | Average 3 | High 2 | High 1 | High 2 |
| PI | 9 | 4 | 3 | 3 |
| Prostate ct | 32 | 58 | 52 | 50 |

**Conclusion**: pain stopped, urination during the first 2 weeks normalized, overall and prostatic functional indicators improved. Follow-up secretion analysis: WBC -4. RBC - none.

Thus the conducted studies confirm the efficacy of the claimed group of inventions. No patient exhibited any signs of toxic, allergic, or any other adverse reactions resulting from the therapeutic effect according to the claimed method with medications obtained by the claimed method.
- Water saturated with radiation from the pharmaceutical preparation has a beneficial effect on the human body (sick and relatively healthy). Radiation from the preparation has a therapeutic effect on patients similar to that of the parent chemical or biological substance. The informational effect is characteristic in that the biological effect is not caused by the electromagnetic field energy, which is negligible, but rather by the informational value thereof. Information affects the regulatory and operational processes of the body. The optimal effect is with 10-7 - 10-6 W/m².
- The overall and specific biofunctional indicators are improved both with radiation and from the usual intake of water with radiation transferred via an intermediate carrier from the parent chemical or biological substance.
- Patients' follow-up for 1 to 6 months clearly demonstrates positive health dynamics and less pronounced clinical manifestations of the disease.
- Transmission of radiation to any distance via the communication media by using the Internet, which enables transmission of properties from pharmaceutical preparations and plants to water, has a good potential and opens up multiple possibilities for the treatment and prevention of many diseases, because radiation is less harmful, less expensive, and offers a more pleasant route of administration.
- The therapeutic effect is achieved with no side effect from the drugs.
- Any preparation in the claimed form can be quickly prepared and used in a remote destination site (provided that communication lines had been previously established)

As a result, the IR and PI functional indicators show improvement and the previously abnormal indicators of the originally affected organ are normalized.

The overall wellbeing of study subjects significantly improved, which confirms that the technical result has been achieved and the technical problem has been solved due to increased safety, expanded range of the drug application and reduced side effects during treatment owing to the sharp cost reduction of the preparations used and the entire treatment.

Thus, a novel dosage form eliminating the direct introduction of any chemical or biological substances into the patient's body and a novel preparation method thereof have been created, and an arsenal of dosage forms and preparation methods thereof has been expanded.

With that, an improved safety, expanded range of the drug usage, and elimination of side effects during treatment are achieved due to the elimination of the direct chemical or pathophysiological effect on the patient's body while using the claimed dosage form, which reproduces main therapeutic properties of the chemical or biological medication and which is obtained via an intermediate neutral carrier comprising and reproducing the characteristic (for the medication) energy-informational low intensity radiation that had been transferred thereupon.

## Claims

1. A dosage form, which is a neutral carrier with low intensity energy-informational electromagnetic radiation transmitted thereupon from the parent chemical or biological substance, which produces the desired pharmacological effect.

2. The dosage form according to claim 1, wherein said neutral carrier is a liquid, patch, gel, suspension, or homeopathic pellets.

3. The dosage form according to claim 2, wherein said liquid is an aqueous medium, such as potable water or saline solution.

4. A method for the preparation of the dosage form according to claim 1, wherein said method provides transmission of the low intensity energy-informational electromagnetic radiation bearing the desired pharmacological effect of the parent chemical, plant-based, or biological drug onto a neutral carrier suitable for interaction with the patient's body.

5. The method according to claim 4, wherein said low intensity energy- informational electromagnetic radiation is transmitted onto the neutral carrier from an intermediate carrier suitable for the storage and transmission of low intensity energy-informational electromagnetic signals.

6. The method according to claim 5, wherein by applying laser radiation or a magnet to the parent chemical or biological substance, the low intensity energy-informational electromagnetic radiation thereof is transmitted to at least one intermediate carrier where it is stored until transmitted to the neutral carrier.

7. The method according to any of claims 5 and 6, wherein transmission of radiation is previously conducted onto one intermediate carrier; the radiation is wirelessly transmitted through communication media to a second intermediate carrier, upon which the neutral carrier is exposed.

8. The method according to any of claims 4 and 5, wherein at least one intermediate carrier is an optical disk, wherein the information is transmitted and read by a laser or by any other method known in the art.

9. The method according to claim 8, wherein transmission of radiation from one intermediate carrier to another is carried out through communication media, such as the Internet, telephones, or power lines.

10. The method according to claim 7, wherein the neutral carrier exposed on the intermediate carrier is a liquid, patch, gel, suspension, or homeopathic pellets.

11. The method according to claim 10, wherein the neutral carrier exposed on the intermediate carrier is an aqueous liquid, such as potable water or saline solution.
